# EUROPÄISCHE PATENTSCHRIFT

(11) **EP 1 181 313 B1**
(45) Veröffentlichungstag und Bekanntmachung des Hinweises auf die Patenterteilung: **09.02.2011**
(21) Anmeldenummer: 00945603.9
(22) Anmeldetag: 02.06.2000
(51) Int. Cl.: C07K 14/045, A61K 39/25, C12N 15/38, C12N 15/63, G01N 33/68

(54) **PEPTIDE ZUR VAKZINIERUNG GEGEN DAS HUMANE CMV**
PEPTIDES FOR VACCINATING AGAINST HUMAN CMV
PEPTIDES SERVANT A LA VACCINATION CONTRE LE CYTOMEGALOVIRUS HUMAIN

(30) Priorität: 04.06.1999 DE 19927039; 07.09.1999 DE 19943702
(43) Veröffentlichungstag der Anmeldung: 27.02.2002
(73) Patentinhaber: Kern, Florian, 10435 Berlin (DE)
(72) Erfinder: KERN, Florian, D-10435 Berlin (DE); VOLK, Hans-Dieter, D-10178 Berlin (DE); REINKE,Petra, D-10178 Berlin (DE); FAULHABER, Nicole, D-47509 Rheurdt (DE); SUREL, Ingolf-Pascal, D-10315 Berlin (DE); KHATAMZAS, Elham, D-10405 Berlin (DE)
(74) Vertreter: von Kreisler Selting Werner
(86) Internationale Anmeldenummer: PCT/DE2000/001854
(87) Internationale Veröffentlichungsnummer: WO 2000/075180

(56) Entgegenhaltungen:
- WO-A-94/00150
- WO-A-98/26074
- WO-A-99/02183
- WO-A-99/19349
- DE-A- 19 619 255
- DATABASE UNITPROT/SWISSPROT 1. Januar 1990 (1990-01-01), gefunden im SWISSPROT Database accession no. P13202 & A. AKRIGG ET AL: "The structure of the major immediate early gene of human cytomegalovirus strain AD169" VIRUS RESEARCH, Bd. 2, 1985, Seiten 107-121,

## Beschreibung

Die Erfindung betrifft Peptide oder Peptidderivate, die zur Vakzinierung gegen das humane Cytomegalievirus (HCMV) geeignet sind oder zur Diagnostik bei Patienten, wobei untersucht werden kann, ob diese Patienten eine Immunantwort gegen HCMV aufbauen oder aufgebaut haben.

Die humanen Cytomegalieviren (HCMV) bilden eine Gruppe artverwandter Viren, die zu den Herpes Viren zählen. (Lutz SCHNEIDER, 1990, Pharmazie, Vol. 135 Nr. 27, 2396 - 2400). Nach einer Erstinfektion verbleiben die Viren latent im Körper. Physischer oder psychischer Stress können zur Reaktivierung von latentem HCMV führen. Das Virus ist ubiquitär. Die Durchseuchung der Population liegt in Mitteleuropa bei 65%. Die zellvermittelte Immunantwort spielt bei der Kontrolle und der Abwehr gegen die HCMV - Infektion eine wesentliche Rolle. Wurden HCMV spezifische CD8⁺ T Zellen von einem Spender auf einen Patienten, der an HCMV leidet, übertragen, so konnte eine Immunantwort gegen die HCMV - Infektion beobachtet werden. (P.D. GREENBERG et al., 1991, Development of a treatment regimen for human cytomegalovirus (CMV) infection in bone marrow transplantation recipients by adoptive transfer of donor - derived CMV - specific T cell clones expanded in vitro. ANN. N. Y. Acad. Sci., Vol.: 636, pp 184 - 195). Unglücklicher sind nur wenige Epitope des HCMV bekannt, welche spezifisch von CD8⁺ T Zellen erkannt werden. Es wird angenommen, daß die Immunantwort wesentlich durch das 55KD Immediate-Early-Protein 1 (IE-1) und das 65KD Lower-Phospho-Matrix-Protein (pp65) dominiert wird (N.J. ALP et al., 1991, Fine specificity of cellular immune responses in humans to human cytomegalovirus immediate - early 1 protein, J. Virol., Vol: 65, pp 4812 - 4820; und E.H. McLAUGHLIN - TAYLOR et al., 1994, Identification of the major late human cytomegalovirus matrix protein pp65 as a target antigen for CD8+ virus - specific cytotoxic T lymphocytes, J. Med. Virol., Vol.: 43, pp 103 - 110; und weiter, M. WILLS et al. ,1996, The human cytotoxic T-lymphocyte (CTL) response to cytomegalovirus is dominated by structural protein pp65: frequency, specificity, and T-cell receptor usage of pp65-specific CTL, J. Virol. 1996, Vol. 70, pp7569-79).

Die Infektion verläuft bei Erwachsenen mit einem funktionstüchtigen Immunsystem unauffällig und zeigt höchstens unspezifische Symptome, wie Abgeschlagenheit und leicht erhöhte Körpertemperatur. Bei immungeschwächten Erwachsenen stehen bei HCMV - Infektionen pulmonale Erkrankungen und Netzhaut-Entzündungen im Vordergrund. Bei AIDS-Patienten verursachen CMV-Infektionen zahlreiche Todesfälle.

Verschiedene Substanzen werden zur Behandlungen gegen das Cytomegalievirus eingesetzt. Foscamet z.B. ist eine antivirale Substanz mit in Zellkulturen nachgewiesener selektiver Aktivität gegen humane Herpes-Viren, wie zum Beispiel Herpes simplex, Varicella zoster, Epstein-Barr und Cytomegalie-Viren, sowie Hepatitis Viren. Die antivirale Wirkung beruht auf einer Hemmung viraler Enzyme, wie DNA-Polymerasen und reversen Transkriptasen. Auf Cytomegalieviren wirkt Foscamet virostatisch, jedoch können die Viren nicht eliminiert werden (Lutz SCHNEIDER, 1991, Neue Arzneistoffe, Vol 136, Nr. 46). Eine weitere Problematik der Cytomegalievirus-Infektion stellt die Notwendigkeit der bisweilen lebenslangen Dauerbehandlung der Patienten dar (insbesondere bei AIDS). Nachteilig ist weiterhin, daß die Cytomegalieviren in den letzten Jahren resistenter gegen die üblicherweise verwendeten antiviralen Substanzen geworden sind (z.B. Stanat et al., 1991, Antimicrob. Agents, Chemother. Vol 35, Nr. 11: 2191 - 2197 und Knox et al., 1991, Lancet, Vol 337: 1292 - 1293).

Die Sequenz von des 55KD Immediate-Early Protein 1 (IE-1) ist beschrieben und definiert in A. AKRIGG, G.W.G. WILKINSON, J.D. ORAM (1985) The structure of the major immediate early gene of human cytomegalovirus strain AD169, Virus. Res., 2:107-121. Die Sequenz des 55KD Immediate-Early Protein 1 ist in der SWISS-PROT Datenbank, European Bioinformatics Institute, unter der Nummer P13202 (=primary accession number) abgelegt. Das 65 KD Lower-Matrix- Phosphoprotein (pp65) wurde beschrieben in B. RUEGER, S. KLAGES, B. WALLA et al. (1987) Primary structure and transcription of the genes coding for the two virion phosphoproteins pp65 and pp71 of human cytomegalovirus, J. Virol. 61:446 - 453. Die Sequenz des 65 KD Lower Matrix Phosphoprotein ist in der SWISS-PROT Datenbank, European Bioinformatics Institute, unter der Nummer P06725 (=primary accession number) abgelegt. Die Sequenzen beider Proteine sind beschrieben in M.S. CHEE, A.T. BANKIER, S. BECKS et al. (1990) Analysis of the protein-coding content of the sequence of human cytomegalovirus strain AD169, Curr. Top. Mircrobiol. Immunol. 154:125 - 169.

WO9826074 beschreibt Fusions-Peptide aus einem IE1-Fragment (insbesondere Fragment 162-175) und einem pp65-Fragment und die Induktion von T-Helferzellen und cytotoxischen T-Zellen. Dabei werden die Epitope durch HLA class II Paraloge DR8, A2 und B35 präsentiert.

Aufgabe der Erfindung ist das Anbieten von Peptiden oder deren Derivaten, die die Produktion von Interferon-γ oder Tumor-Nekrose-Faktor-α (TNF-α) in CD8⁺ T Zellen, insbesondere von mit HCMV immunisierten Personen mit geeignetem HLA-Typ, induzieren. Diese Peptide und deren Derivate sind als Mittel zur Vakzinierung geeignet. Ebenso sind sie zur Diagnostik geeignet, um feststellen zu können, ob bei einer Person eine gegen HCMV gerichtete zelluläre Immunantwort vorhanden ist, und um diese zu quantifizieren.

Die Aufgabe wird gelöst durch

### (1) Peptide bestehend aus der Sequenz (I)

R_{N} - Ala Arg Ala Lys Lys Asp Glu Leu Arg Arg Lys Met Met Tyr Met- R_{c} (I) oder Peptidderivate davon, dabei steht

R_{N} für -H oder eine Amino-Schutzgruppe oder eine weitere Aminosäure außerhalb des Peptids oder Peptidderivats,

R_{c} für -OH oder eine Carboxyl-Schutzgruppe oder eine weitere Aminosäure außerhalb des Peptids oder Peptidderivats,
wobei die Peptidderivate eine Deletion, Insertion oder Substituierung von ein, zwei oder drei Aminosäuren der Sequenz (I) aufweisen, oder die Sequenz (I) bis auf neun zusammenhängende Aminosäuren gekürzt wird, wobei die Deletion N-terminal und/oder C-terminal ist, und
wobei die Peptidderivate im wesentlichen die Funktion des Peptides mit der Sequenz (I) oder eines der folgenden Peptide
Glu Leu Arg Arg Lys Met Met Tyr Met
Asp Glu Leu Arg Arg Lys Met Met Tyr
Asp Glu Leu Arg Arg Lys Met Met Tyr Met oder
Asp Glu Leu Arg Arg Lys Met Met Tyr Met
(jede der vorherigen Sequenzen = Referenzsequenz) besitzen, in CD8⁺ T Zellen, insbesondere von mit HCMV immunisierten Personen mit geeignetem HLA-Typ, die Produktion von Interferon-γ oder TNF-α zu induzieren; und

### (2) Peptide bestehend aus der Sequenz (II)

R_{N} - Glu Phe Cys Arg Val Leu Cys Cys Tyr Val Leu Glu Glu Thr Ser- R_{c} (II) oder Peptidderivate davon, dabei steht

R_{N} für -H oder eine Amino-Schutzgruppe oder eine weitere Aminosäure außerhalb des Peptids oder Peptidderivats,

R_{c} für -OH oder eine Carboxyl-Schutzgruppe oder eine weitere Aminosäure außerhalb des Peptids oder Peptidderivats,
wobei die Peptidderivate eine Deletion, Insertion oder Substituierung von ein, zwei oder drei Aminosäuren der Sequenz (II) aufweisen, oder die Sequenz (II) bis auf neun zusammenhängende Aminosäuren gekürzt wird, wobei die Deletion N-terminal und/oder C-terminal ist, und
wobei die Peptidderivate im wesentlichen die Funktion des Peptides mit der Sequenz (II) oder eines der folgenden Peptide
Cys Arg Val Leu Cys Cys Tyr Val Leu
Arg Val Leu Cys Cys Tyr Val Leu Glu
Val Leu Cys Cys Tyr Val Leu Glu Glu
(jede der vorherigen Sequenzen = Referenzsequenz) besitzen, in CD8⁺ T Zellen, insbesondere von mit HCMV immunisierten Personen mit geeignetem HLA-Typ, die Produktion von Interferon-γ oder TNF-α zu induzieren.

Bevorzugte Peptide und Peptidderivate von (I) und (II) weisen die folgenden Sequenzen auf:
R_{N} - Ala Arg Ala Lys Lys Asp Glu Leu Arg Arg Lys Met Met Tyr Met- R_{c}
R_{N} - Asp Glu Leu Arg Arg Lys Met Met Tyr Met- R_{c}
R_{N} - Glu Leu Arg Arg Lys Met Met Tyr Met- R_{c}
R_{N} - Asp Glu Leu Arg Arg Lys Met Met Tyr - R_{c}
R_{N} - Asp Glu Leu Arg Arg Lys Met Met Tyr Met - R_{c}
R_{N} - Glu Phe Cys Arg Val Leu Cys Cys Tyr Val Leu Glu Glu Thr Ser- R_{c}
R_{N} - Cys Arg Val Leu Cys Cys Tyr Val Leu - R_{c}
R_{N} - Arg Val Leu Cys Cys Tyr Val Leu Glu - R_{c}
R_{N} - Val Leu Cys Cys Tyr Val Leu Glu Glu - R_{c}.

Die Erindung betrifft weiterhin
(3) die Verwendung eines Peptides oder Peptidderivates nach (1) und (2) zur Herstellung eines Medikaments zur Vakzinierung gegen HCMV Infektionen;
(4) die Verwendung eines Peptides oder Peptidderivates nach (1) und (2) zur Herstellung eines Diagnostikums zur Identifizierung einer Reaktion des zellulären Immunsystems gegen HCMV;
(5) die Verwendung eines Peptides oder Peptidderivates nach (1) und (2) zur Herstellung eines Diagnostikums zur Quantifizierung einer Reaktion des zellulären Immunsystems gegen HCMV;
(6) eine DNA, welche für eine der Aminosäuresequenzen und deren Derivate nach (1) oder (2) kodiert;
(7) Vektoren oder Plasmide, in welche die DNA nach (6) eingebaut ist; und
(8) ein Medikament, umfassend eine DNA, Plasmid oder Vektor nach (6) oder (7).

Ein geeigneter HLA - Typ (Human Leukocyte Antigen) ist eine Kombination von MHC - Klasse I Molekülen (Major Histocompatibility Complex), von denen eines oder mehrere die Präsentation der beschriebenen Peptide ermöglichen. Nur wenn ein geeigneter HLA - Typ vorliegt, kann ein bestimmtes Peptid auch präsentiert werden, und nur dann kann es zur Stimulation kommen. Geeignete Referenz für den MHC an sich und die Antigenerkennung von T Zellen: Abdul K. ABBAS Cellular and Molecular Immunology /A.K. Abbase A.H. Lichtman, J.S. Prober, Chapter 5 and 6, 1991, W.B. SAUNDERS, Philadephia, ISBN 0-7216-3032-4

Viele Patienten, die eventuell vakziniert werden können, sind bereits infiziert. Bei diesen Patienten handelt es sich somit um eine Verstärkung der Immunantwort ("Boostern*). Die Infektion kann latent sein oder aktiv auftreten. Auch Personen mit latentem Virus sind als "infiziert" anzusehen.

Bevorzugte Referenzsequenz ist:
Cys Arg Val Leu Cys Cys Tyr Val Leu.

Die einzelnen Aminosäuren haben in den jeweiligen Positionen unterschiedliche Bevorzugung.
Der Vergleich der verschiedenen Aminosäuren erfolgt durch Austausch einer Aminosäure in einer definierten Position bei gleichzeitiger Beibehaltung der anderen Aminosäuren in den restlichen Positionen. Generell sind auch mehrfache Substituierungen möglich.

Die Funktionen der Peptide oder Peptidderivate werden wesentlich verändert, wenn Substituenten gewählt werden, die bei der Substituierung weniger konservativ als die im folgenden erwähnten Aminosäuren sind. So sind die Substituenten Gly und Ser der Aminosäure Ala ähnlich, weiterhin ist der Substituent Lys der Aminosäure Arg ähnlich. Die Substituenten Gln und His sind der Aminosäure Asn ähnlich, der Substituent Glu ist der Aminosäure Asp ähnlich, der Substituent Ser ist der Aminosäure Cys ähnlich, der Substituent Asn ist der Aminosäure Gln ähnlich, der Substituent Asp ist der Aminosäure Glu ähnlich, der Substituent Thr ist der Aminosäure Ser ähnlich, der Substituent Ser ist der Aminosäure Thr ähnlich, der Substituent Tyr ist der Aminosäure Trp ähnlich, die Substituenten Ala und Pro sind der Aminosäure Gly ähnlich, die Substituenten Asn und Gln sind der Aminosäure His ähnlich, die Substituenten Leu und Val sind der Aminosäure Ile ähnlich, die Substituenten Ile und Val sind der Aminosäure Leu ähnlich, die Substituenten Arg, Gln und Glu sind der Aminosäure Lys ähnlich, die Substituenten Leu, Tyr und Ile sind der Aminosäure Met ähnlich, die Substituenten Met, Leu und Tyr sind der Aminosäure Phe ähnlich, die Substituenten Trp und Phe sind der Aminosäure Tyr ähnlich, und die Substituenten Ile und Leu sind der Aminosäure Val ähnlich. Derartige wesentlichen Veränderungen lassen sich durch Substituierungen mit Aminosäuren erzielen, die sich mehr in ihrer Struktur und in den funktionellen Gruppen unterscheiden. Wesentliche Veränderungen wirken sich dahingehend aus, daß die dreidimensionale Struktur deutlich verändert wird und/oder daß zum Beispiel die Faltblatt - Struktur oder die helikale Struktur beeinflußt wird. Auch Wechselwirkungen der Ladungen und der hydrophoben Ketten sind bei den Veränderungen zu beachten. Derartige Analysen über Substituierungen lassen sich leicht bewerkstelligen. Dabei wird je eine Aminosäure in einer Position gegen bevorzugt Alanin oder eine weitere Aminosäure ausgetauscht. Nach der Synthese des modifizierten Proteins wird die Funktion des veränderten Peptids gemessen. Die Funktionen und deren Messung sind in den Beispielen dargestellt. Auch mehrfache Substituierungen sind möglich.

### Definitionen

Die im Text verwendeten **Abkürzungen** sind durch die Regeln bestimmt, die von der IUPAC-IUB Kommission für biochemische Nomenklatur festgelegt worden sind (Biochemistry 11: 1726 (1972) und Biochem. J. 219: 345 (1984)). Folgende übliche Abkürzungen werden verwendet: Ala = A = Alanin; Arg = R= Arginin; Asn =N = Asparagin; Asp = D = Asparaginsäure; Cys = C = Cystein; Gln = Q = Glutamin; Glu = E = Glutaminsäure; Gly = G = Glycin; His = H = Histidin; Ile = I = Isoleucin; Leu = L = Leucin; Lys = K = Lysin;. Met = M = Methionin; Phe = F = Phenylalanin; Pro = P = Prolin; Ser = S = Serin; Thr =T = Threonin; Trp = W = Tryptophan; Tyr = Y = Tyrosin und Val = V = Valin.

Die **Schutzgruppe** des Restes R_{N} kann bestehen aus:
Alkyl-, Aryl-, Alkylaryl-, Aralkyl-, Alkylcarbonyl- oder Arylcarbonylgruppen mit 1 bis 10 Kohlenstoffatomen, bevorzugt sind Naphthoyl-, Naphthylacetyl-, Naphthylpropionyl-, Benzoylgruppe oder einer Acylgruppe mit 1 bis 7 Kohlenstoffatomen.

Die **Schutzgruppe** des Restes R_{C} kann bestehen aus: .
Eine Alkoxy- oder Aryloxygruppe mit 1 bis 10 Kohlenstoffatomen oder aus einer Aminogruppe.
Weitere **Schutzgruppen -** sowohl für R_{N} und R_{C} - sind in Houben-Weyl (1974) Georg Thieme Verlag, 4. Auflage beschrieben. Die Beschreibung der Schutzgruppen in der zitierten Literaturangabe ist Teil der Offenbarung.

Die aufgeführten **Aminosäuren** sind natürliche oder künstliche Aminosäuren. Die künstlichen Aminosäuren sind in Houben-Weyl (1974) Georg Thieme Verlag, 4. Auflage beschrieben. Dabei ist der Austausch einer beschriebenen Aminosäure durch eine weitere Aminosäure, die zur Gruppe der natürlichen oder künstlichen Aminosäuren gehört, leicht möglich. Aufgrund des Testsystems und Vergleichs mit einem der zuvor ausdrücklich aufgeführten Peptide aus der Gruppe der Referenzsequenzen ist es leicht möglich, herauszufinden, ob eine Wirkung vorliegt, die gleich oder ähnlich der Wirkung der gefundenen erfindungsgemäßen Substanzen ist. Unter den Schutzumfang fallen auch alle D-Aminosäuren und alle Aminosäuren, die künstlich herstellbar sind. (Houben-Weyl). Für den Fachmann ist es ein leichtes, unter Beibehaltung der Funktionen, die im Test überprüfbar sind, die molekulare Struktur unter Beibehaltung der wesentlichen Bausteine abzuwandeln. Auch diese funktionell gleichwirkenden Moleküle fallen unter den Begriff der Peptidderivate.

### Vorteile

Die erfindungsgemäßen Peptide oder Peptidderivate ermöglichen es, gezielt Vakzinen gegen eine Infektion mit humanen Cytomegalievirus herzustellen. Auch können die Peptide und Peptidderivate als Diagnostika verwendet werden, um zu sehen, ob die zu untersuchenden Patienten, insbesondere solche mit geeignetem HLA-Typ, CD8+ T Zellen besitzen, die durch die erfindungsgemäßen Substanzen zur Produktion von Interferon-γ oder TNF-α (Tumor - Nekrose - Faktor alpha) induziert werden.

### Bevorzugte Ausführungsformen

Bevorzugt sind Nonamere, die darin bestehen, daß eine längere Sequenz, wie sie zuvor ausdrücklich genannt worden ist oder deren Derivate bis auf neun zusammenhängende Aminosäuren gekürzt werden. Dabei kann die Deletion N - terminal und / oder C - terminal sein. Wesentlich ist dabei, daß die Funktion von einem Peptid aus der Gruppe der Referenzsequenzen im Wesentlichen erfüllt wird. Daß Nonamere sehr potente Stimulatoren von CD8+ T Zellen sind, ist dadurch bedingt, das MHC - Klasse I präsentierte Peptide typischer Weise eine Länge von neun Aminosäuren aufweisen. (K.O. FALK et al. (1991) Allele - specific motifs revealed by sequencing of self - peptides eluted from MHC molecules, Nature, Vol.: 351, pp 290 - 296 und H.G. RAMMENSEE et al. (1999) An Internet Database for MHC Ligands and Peptide Motifs, http://134.296.221/scripts/hlaserver.dll/home.htm)

Ebenso ist es möglich, die zuvor genannten Nonamere mit weiteren Aminosäuren peptidisch zu verbinden, so daß Sequenzen von mindestens 10 Aminosäuren entstehen. Diese Sequenzen haben ebenfalls die Funktion, in CD8⁺ T Zellen die Produktion von Interferon-γ oder TNF-α zu induzieren. Somit zählen zum Schutzumfang der Erfindung auch die um mindestens eine Aminosäure verlängerten Sequenzen von Nonameren, welche die Funktion aufweisen, in CD8⁺ T Zellen, insbesondere von mit HCMV immunisierten Personen mit geeignetem HLA-Typ, die Produktion von Interferon-γ oder TNF-α zu induzieren. Für die verlängerten Nonamere ist wesentlich, daß sie ebenfalls die Funktion besitzen, in CD8⁺ T Zellen, insbesondere von mit HCMV immunisierten Personen mit geeignetem HLA-Typ, die Produktion von Interferon-γ oder TNF-α zu induzieren.

### Herstellung der Peptide

Weiterhin sind die erfindungsgemäßen Peptide oder Peptidderivate leicht herstellbar. Derart kurze Peptide oder Peptidderivate können mittels einer Technik hergestellt werden, die den Fachleuten im Bereich der Peptidsynthese bekannt ist. Eine Zusammenfassung vieler dieser Techniken können bei J.M. STEWART and J.D. YOUNG, San Francisco, 1969; und J. MEIERRHOFER, Hormonal Proteins and Peptides, Vol. 2 p 46, Academic Press (New York), 1973 für die Festphasen-Methode und E. SCHRODER and K.LUBKE, The Peptides, Vol. 1, Academic Press (New York) 1965 für die Flüssigphasen-Methode nachgelesen werden. Die Schritte der Synthese sind in den EP-A 0 097 031 beschrieben. Die allgemeinen Verfahrensschritte aus den europäischen Publikationen lassen sich analog auf die Synthese der hier beschriebenen erfindungsgemäßen Peptide oder Peptidderivate übertragen. Weitere Literatur zu der Festphasensynthese sind: Solid Phase Synthesis, E. ATHERTON and R.C. SHEPPARD (1989) IRL Press, ISBN 1-85221-133-4 and Amino Acid and Peptide Synthesis, J. JONES, Oxford Science Publication (1992) ISBN 0-19-855668-3.

### Weitere Ausführungsformen bezüglich der Reste

Neben der Abwandlung der Aminosäure-Sequenz der erfindungsgemäßen Peptide oder Peptidderivate ist auch eine Variation der Reste R_{N} und R_{c} möglich. Die Reste brauchen jedoch die Funktion nicht zu beeinflussen. Dennoch lassen sich durch Schutzgruppen Parameter wie Stabilität, pH-Abhängigkeit, biologische Abbaubarkeit und Interaktionen mit dem nativen Teil des Fusionsproteins deutlich beeinflussen.

Bevorzugt sind erfindungsgemäße Peptide oder Peptidderivate, bei denen der Rest
R_{N} für -H oder eine Amino-Schutzgruppe
und
R_{c} für -OH oder eine Carboxyl-Schutzgruppe steht.

Bevorzugter sind erfindungsgemäße Peptide oder Peptidderivate, bei denen die Reste stehen:
R_{N} für -H oder eine Acylgruppe
und
R_{c}für -OH oder Amino-Gruppe.

Am meisten bevorzugt sind erfindungsgemäße Peptide oder Peptidderivate, bei denen die Reste stehen:
R_{N} für -H
und
R_{c} für -OH.

### Herstellungsverfahren der erfindungsgemäßen Peptide

Die Erfindung umfaßt weiterhin die Herstellung der erfindungsgemäßen Peptide oder Peptidderivate, wobei eine N-α geschützte ω-Amino-α-aminosäure mit einem Dialdehyd in Gegenwart eines Reduktionsmittels umgesetzt und anschließend die Schutzgruppen der Seitenketten und gegebenenfalls die Schutzgruppen des N-Terminus und/oder des C-Terminus abgespalten werden.

Ebenfalls umfaßt die Erfindung ein Verfahren, bei dem die erfindungsgemäßen Peptide oder Peptidderivate hergestellt werden, indem die Aminosäuren in einer homogenen Phase oder nach der Festphasen - Methode kondensiert werden,
wobei das Carboxyl - Ende einer zu koppelnden Aminosäure, deren Aminogruppen und gegebenenfalls funktionellen Gruppen der Seitenkette eine Schutzgruppe tragen, mit dem freien Amino - Ende der zu koppelnden Aminosäure oder des zu koppelnden Peptids in Gegenwart eines Kondensationsreagenzes reagiert,
und
im Falle einer nicht-endständigen Aminosäure anschließend die α-Amino-Schutzgruppe der gekoppelten Aminosäure abgespalten wird und weitere Aminosäuren an die zu synthetisierende Peptid-Kette nach den zuvor beschriebenen beiden Schritten gekoppelt werden
oder
im Falle einer endständigen Aminosäure gegebenenfalls anschließend die α-Amino-Schutzgruppe der gekoppelten Aminosäure abgespalten wird
und
nach Kopplung der letzten Aminosäure im Falle der Festphasen-Methode das Peptid oder Peptidderivat von der Festphase abgespalten wird.

### Verwendung als Medikament

Die erfindungsgemäßen Peptide oder Peptidderivate sind geeignet als Medikament oder Diagnostikum eingesetzt zu werden.

Am meisten bevorzugt ist die Verwendung eines Peptids oder Peptidderivates zur Herstellung eines Medikaments zur Vakzinierung gegen das humane Cytomegalievirus.

Ebenfalls vorteilhaft ist die Verwendung eines erfindungsgemäßen Peptids oder Peptidderivates zur Herstellung eines Diagnostikums zur Identifizierung einer zellulären Immunantwort gegen HCMV. Dabei können T-Zellen des Patienten in vitro mit den erfindungsgemäßen Peptiden und Peptidderivaten stimuliert werden. Wird dabei die Interferon-γ oder TNF-α Produktion in CD8⁺ T Zellen induziert, so ist eine Immunantwort gegen HCMV nachgewiesen. Führt diese Stimulation nicht zur Induktion von Interferon-γ oder TNF-α in CD8⁺ T-Zellen einer mit HCMV immunisierten Person mit geeignetem HLA-Typ, kann dies bedeuten, daß diese Person keine CD8⁺ T-Zellantwort gegen das HCMV aufgebaut hat oder eine bestehende CD8⁺ T-Zellantwort sich nicht gegen das zur Stimulation verwendete Epitop richtet.

Besonders vorteilhaft ist der Einsatz eines erfindungsgemäßen Peptids oder Peptidderivates zur Herstellung eines Diagnostikums zur Identifizierung einer Immunantwort gegen HCMV bei immungeschwächten Personen.

Die mittels der Induktion von INF-γ oder TNF-α in CD8+ T-Zellen identifizierte Immunantwort gegen HCMV, kann quantifiziert werden, indem die Anzahl der CD8+ T-Zellen bestimmt wird, in welchen es zur induktion dieser Zytokine gekommen ist. Diese Anzahl kann absolut (pro Volumen des Ausgangsmaterials) oder relativ (bezogen auf beispielsweise alle CD8+ T-Zellen) angegeben werden. Diese Quantifizierung kann beispielsweise durchflußzytometrisch oder mittels eines geeigneten anderen Verfahrens erfolgen.

Als Medikament sind die erfindungsgemäßen Peptide oder Peptidderivate bevorzugt, wenn sie eine Zusammensetzung mit pharmakologisch verträglichen Hilfs- und Trägerstoffen bilden. Derartige Hilfs- und Trägerstoffe sind in Remington's Pharmaceutical Science, 15th ed. Mack Publishing Company, East Pennsylvania (1980) beschrieben. Die Zusammensetzungen können nach bekannten Verfahren hergestellt werden.

Vorteilhaft ist die Verwendung der Peptide oder Peptidderivate zur Herstellung eines Medikaments zur Behandlung, wenn Sie zum Beladen dendritischer Zellen verwendet werden, welche anschließend einem Patienten als Medikament verabreicht werden. Vorteilhafter ist es, wenn die Peptide oder Peptidderivate zum Beladen HLA-identischer oder -teilidentischer dendritischer Zellen verwendet werden, welche anschließend einem Patienten als Medikament verabreicht werden.

Die Verwendung von peptidbeladenen dendritischen Zellen als Vakzine wird bei Brugger et al., Ann. N. Y. Acad. Sci., Vol. 872, pp 363-371, beschrieben.

Die erfindungsgemäßen Peptide oder Peptidderivate besitzen pharmakologische Eigenschaften und sind deshalb als pharmazeutischer Wirkstoff oder Diagnostikum, insbesondere als Vakzine oder Diagnostikum verwendbar. Die Erfindung umfaßt ebenfalls ein Arzneimittel, das die erfindungsgemäßen Peptide oder Peptidderivate enthält.

Die Versuchsergebnisse der *in vitro* Testung zeigen, daß die erfindungsgemäßen Peptide oder Peptidderivate als Arzneimittel oder zur medizinischen Behandlung verwendet werden können. Diese Versuchsergebnisse lassen sich von dem *in vitro* Testsystem auf ein *in vivo* System problemlos übertragen.

Die Erfindung liefert weiterhin
(i) die Verwendung von erfindungsgemäßen Peptiden oder Peptidderivaten zur Herstellung eines Medikaments als Vakzine gegen Infektionen mit HCMV;
(ii) eine pharmakologische Zusammensetzung als Vakzine gegen Infektionen des HCMV, welche als Behandlung oder Prophylaxe die erfindungsgemäße Peptide oder Peptidderivate und wenigstens einen pharmazeutischen Hilfs- und / oder Trägerstoff umfaßt.

Für die therapeutische Wirkung sind unterschiedliche Dosen geeignet. Sie hängen beispielsweise von den verwendeten Salzen, vom Wirt, von der Art der Verabreichung und von der Art und der Schwere der zu behandelnden Zustände ab.

Auch sind Kombinationen der erfindungsgemäßen Peptide oder Peptidderivate möglich.

Die Erfindung umfaßt weiterhin DNA (Desoxyribonucleic acid = Desoxyribonucleinsäure), welche für eine der zuvor genannten Aminosäuresequenzen und deren Derivate kodiert.
Diese DNA kann sinnvoller Weise in Vektoren oder Plasmide eingebaut sein. Solche Vektoren sind geeignet, in menschliche Zellen einzudringen und dort mit der Proteinbiosynthese zu beginnen. In dieser Form kann der Proteinbiosynthese - Apparat des Menschen verwendet werden, um die gewünschten Peptide zu synthetisieren und zu sezernieren.
Derartige Vektoren werden mit anders kodierenden DNA beschrieben in D. Salmon-Ceron et al. (1999) AIDS Res Hum Retroviruses Vol 15/7, pp 633 - 645 und F. DORNER et al. (1999) Ann Med. Vol 31/1, pp 51 - 60 und G. FERRARI et al. (1997) Blood, Vol 90, pp 2406 - 2416 und K. MOLLING (1998) Z. Arztl. Fortbild. Qualitätssich Vol 92, pp 681 - 683 und M. Giese (1998), Virus Genes Vol 17, pp 219 - 232

### Beispiele

### Methoden

Mit Zitrat versetztes Blut wurde von anti-HCMV-IgG-seropositiven Blutspendern erhalten, die einen definierten HLA - Typ besitzen. Es folgte eine Ficoll - Paque Dichte Zentrifugation. Die Zellen wurden mit sterilem PBS gewaschen. Sie wurden in RPMI 1640 resuspendiert, welches 0,1 % BSA und 2 mM Glutamin enthielt. Die Zellen wurden auf 10⁷ Zellen pro ml eingestellt. 200 µl der Zell - Suspension und der Peptid - Lösung (10µg pro ml in RPMI / BSA) wurden in Cellstar ^{R} Polystyren - Röhrchen gefüllt und in einem Inkubator gelagert.
Nach einer Stunde wurden 1600 µl RPMI 1640 hinzugegeben, welches 12,5% FCS, 50 mM Glutamin und 12,5 µg pro ml Brefeldin A enthielt. Nach fünf weiteren Stunden wurden die Zellen mit kaltem PBS gewaschen, resuspendiert in PBS, mit 1 mM EDTA, inkubiert für 10 weitere Minuten bei 37°C und erneut mit kaltem PBS gewaschen. Nach einer Oberflächenmarkierung mit monoklonalen Antikörpern über 30 Minuten bei 4°C im Dunkeln wurden die Zellen in PBS, das 4 % Paraformaldehyd enthielt, über 4 Minuten bei 37 °C fixiert und anschließend mit PBS gewaschen. Die Zellen wurden dann mittels der Permeabilisierungslösung von Becton Dickinson (Heidelberg) permeabilisiert und anschließend nochmals mit PBS gewaschen.
Im Anschluß an die folgende intrazelluläre Markierung mit monoklonalen Antikörpern gegen Interferon-γ und/oder TNF-α wurden die Zellen erneut in PBS gewaschen und mit einem FACScalibur ^{R} Durchfluß - Zytometer (Becton Dickinson) unter Verwendung einer CellQuest^{™} Software analysiert. Nicht stimulierte Proben wurden als Kontrolle eingesetzt.

### Ergebnisse:

Die erfindungsgemäßen Substanzen zeigten eine Stimulierung der CD8⁺ T Zellen. Diese Substanzen sind daher als Vakzine geeignet. Weiterhin sind sie geeignet, als Diagnostikum eingesetzt zu werden, wobei die Zellen identifiziert werden, die auf HCMV reagieren können. Die Unfähigkeit oder Fähigkeit eines Patienten, auf HCMV reagieren zu können, oder eine bereits stattgefundene Immunisierung mit HCMV werden durch diese Form der Diagnostik festgestellt. Die Stimulierung der CD8+ T Zellen wurde durch das Vorhandensein von intrazelluär zurückgehaltenem Interferon-γ oder Tumor - Nekrose - Faktor - α(TNFα) nachgewiesen.

### Herstellung der Peptide

Die Synthese von Peptiden oder Peptidderivaten ist an einem Multiplen Peptidsynthesizer (MPS) AMS 422 von ABIMED (Langenfeld) ausführbar (H. GAUSEPOHL et al. (1992) Peptide Research 5/6: 315 - 320).
Für die Peptidsynthese können als feste Träger gehärtete Zellulose (Whatman 540; Katalog Nummer 1540917) der Firma Whatman (Maidstone, Großbritannien) und Polystyrolharz Tenta Gel SRAM (Kapazität 0,25 meq/g) der Firma Rapp Polymere (Tübingen, Deutschland) benutzt werden.
Ausführlich ist die Festphasen Peptidsynthese beschrieben in Rudolf Volkmer- Engert, Berit Hoffmann and Jens Schneider-Mergener, (1997) Stable Attachement of the HMB-Linker to Continuous Cellulose Membranes for Parallel Solid Phase Spot Syntheses, Tetrahedron Letter, Vol. 38,6; pp 1029 - 1032.

### Sequenzprotokoll:

**(1) ALLGEMEINE ANGABEN**
**(i) ANMELDER:**

| | | |
|---|---|---|
| (A) | NAME: | Florian **KERN** |
| (B) | STRASSE: | Wolliner Straße 9 |
| (C) | ORT: | 10435 BERLIN |
| (E) | LAND: | **DEUTSCHLAND** |
| (F) | POSTLEITZAHL: | 10435 |

**(ii) BEZEICHNUNG DER ERFINDUNG: Peptide zur Vakzinierung gegen das humane CMV**
**(iii) ANZAHL DER SEQUENZEN: 33 Sequenzprotokolle**
**(iv) COMPUTERLESBARE FASSUNG**

| | | |
|---|---|---|
| (A) | DATENTRÄGER: | **DISKETTE** |
| (B) | COMPUTER: | **Pentium/INTEL** |
| (C) | BETRIEBSSYSTEM: | **WINDOWS** |
| (D) | SOFTWARE: | **WINWORD;** |

**(v) DATEN DER JETZIGEN ANMELDUNG:** Prioritätsbelege vom 4.6.1999 (199 27 039.2) und 7.9.1999 (199 43 702.5). Anmeldetag dieser Anmeldung 2.6.2000
(2) ANGABEN ZU SEQ. ID. NO: 1:
(i) SEQUENZKENNZEICHEN

| | |
|---|---|
| SEQUENZLÄNGE: | 15 Aminosäuren |
| ART DER SEQUENZ: | Aminosäure-Sequenz |
| MERKMAL: | Vakzine gegen HCMV |

SEQUENZBESCHREIBUNG: SEQ. ID. NO: 1:
Gln Thr Met Leu Arg Lys Glu Val Asn Ser Gln Leu Ser Leu Gly
(2) ANGABEN ZU SEQ. ID. NO: 2:
(i) SEQUENZKENNZEICHEN

| | |
|---|---|
| SEQUENZLÄNGE: | 15 Aminosäuren |
| ART DER SEQUENZ: | Aminosäure-Sequenz |
| MERKMAL: | Vakzine gegen HCMV |

SEQUENZBESCHREIBUNG: SEQ. ID. NO: 2:
Ala Arg Ala Lys Lys Asp Glu Leu Arg Arg Lys Met Met Tyr Met
(2) ANGABEN ZU SEQ. ID. NO: 3:
(i) SEQUENZKENNZEICHEN

| | |
|---|---|
| SEQUENZLÄNGE: | 10 Aminosäuren |
| ART DER SEQUENZ: | Aminosäure-Sequenz |
| MERKMAL: | Vakzine gegen HCMV |

SEQUENZBESCHREIBUNG: SEQ. ID. NO: 3:
Asp Glu Leu Arg Arg Lys Met Met Tyr Met
(2) ANGABEN ZU SEQ. ID. NO: 4:
(i) SEQUENZKENNZEICHEN

| | |
|---|---|
| SEQUENZLÄNGE: | 15 Aminosäuren |
| ART DER SEQUENZ: | Aminosäure-Sequenz |
| MERKMAL: | Vakzine gegen HCMV |

SEQUENZBESCHREIBUNG: SEQ. ID. NO: 4:
Glu Leu Arg Arg Lys Met Met Tyr Met Cys Tyr Arg Asn Ile Glu
(2) ANGABEN ZU SEQ. ID. NO: 5:
(i) SEQUENZKENNZEICHEN

| | |
|---|---|
| SEQUENZLÄNGE: | 15 Aminosäuren |
| ART DER SEQUENZ: | Aminosäure-Sequenz |
| MERKMAL: | Vakzine gegen HCMV |

SEQUENZBESCHREIBUNG: SEQ. ID. NO: 5:
Glu Phe Cys Arg Val Leu Cys Cys Tyr Val Leu Glu Glu Thr Ser
(2) ANGABEN ZU SEQ. ID. NO: 6:
(i) SEQUENZKENNZEICHEN

| | |
|---|---|
| SEQUENZLÄNGE: | 9 Aminosäuren |
| ART DER SEQUENZ: | Aminosäure-Sequenz |
| MERKMAL: | Vakzine gegen HCMV |

SEQUENZBESCHREIBUNG: SEQ. ID. NO: 6:
Cys Arg Val Leu Cys Cys Tyr Val Leu
(2) ANGABEN ZU SEQ. ID. NO: 7:
(i) SEQUENZKENNZEICHEN

| | |
|---|---|
| SEQUENZLÄNGE: | 9 Aminosäuren |
| ART DER SEQUENZ: | Aminosäure-Sequenz |
| MERKMAL: | Vakzine gegen HCMV |

SEQUENZBESCHREIBUNG: SEQ. ID. NO: 7:
Arg Val Leu Cys Cys Tyr Val Leu Glu
(2) ANGABEN ZU SEQ. ID. NO: 8:
(i) SEQUENZKENNZEICHEN

| | |
|---|---|
| SEQUENZLÄNGE: | 9 Aminosäuren |
| ART DER SEQUENZ: | Aminosäure-Sequenz |
| MERKMAL: | Vakzine gegen HCMV |

SEQUENZBESCHREIBUNG: SEQ. ID. NO: 8:
Val Leu Cys Cys Tyr Val Leu Glu Glu
(2) ANGABEN ZU SEQ. ID. NO: 9:
(i) SEQUENZKENNZEICHEN

| | |
|---|---|
| SEQUENZLÄNGE: | 9 Aminosäuren |
| ART DER SEQUENZ: | Aminosäure-Sequenz |
| MERKMAL: | Vakzine gegen HCMV |

SEQUENZBESCHREIBUNG: SEQ. ID. NO: 9:
Glu Leu Arg Arg Lys Met Met Tyr Met
(2) ANGABEN ZU SEQ. ID. NO: 10:
(i) SEQUENZKENNZEICHEN

| | |
|---|---|
| SEQUENZLÄNGE: | 9 Aminosäuren |
| ART DER SEQUENZ: | Aminosäure-Sequenz |
| MERKMAL: | Vakzine gegen HCMV |

SEQUENZBESCHREIBUNG: SEQ. ID. NO: 10:
Asp Glu Leu Arg Arg Lys Met Met Tyr
(2) ANGABEN ZU SEQ. ID. NO: 11:
(i) SEQUENZKENNZEICHEN

| | |
|---|---|
| SEQUENZLÄNGE: | 15 Aminosäuren |
| ART DER SEQUENZ: | Aminosäure-Sequenz |
| MERKMAL: | Vakzine gegen HCMV |

SEQUENZBESCHREIBUNG: SEQ. ID. NO: 11:
Cys Asn Glu Asn Pro Glu Lys Asp Val Leu Ala Glu Leu Val Lys
(2) ANGABEN ZU SEQ. ID. NO: 12:
(i) SEQUENZKENNZEICHEN

| | |
|---|---|
| SEQUENZLÄNGE: | 15 Aminosäuren |
| ART DER SEQUENZ: | Aminosäure-Sequenz |
| MERKMAL: | Vakzine gegen HCMV |

SEQUENZBESCHREIBUNG: SEQ. ID. NO: 12:
Leu Val Lys Gln Ile Lys Val Arg Val Asp Met Val Arg His Arg
(2) ANGABEN ZU SEQ. ID. NO: 13:
(i) SEQUENZKENNZEICHEN

| | |
|---|---|
| SEQUENZLÄNGE: | 15 Aminosäuren |
| ART DER SEQUENZ: | Aminosäure-Sequenz |
| MERKMAL: | Vakzine gegen HCMV |

SEQUENZBESCHREIBUNG: SEQ. ID. NO: 13:
Ala Ala Asn Lys Leu Gly Gly Ala Leu Gln Ala Lys Ala Arg Ala
(2) ANGABEN ZU SEQ. ID. NO: 14:
(i) SEQUENZKENNZEICHEN

| | |
|---|---|
| SEQUENZLÄNGE: | 10 Aminosäuren |
| ART DER SEQUENZ: | Aminosäure-Sequenz |
| MERKMAL: | Vakzine gegen HCMV |

SEQUENZBESCHREIBUNG: SEQ. ID. NO: 14:
Asp Glu Leu Arg Arg Lys Met Met Tyr Met
(2) ANGABEN ZU SEQ. ID. NO: 15:
(i) SEQUENZKENNZEICHEN

| | |
|---|---|
| SEQUENZLÄNGE: | 15 Aminosäuren |
| ART DER SEQUENZ: | Aminosäure-Sequenz |
| MERKMAL: | Vakzine gegen HCMV |

SEQUENZBESCHREIBUNG: SEQ. ID. NO: 15:
Val Thr Ser Asp Ala Cys Met Met Thr Met Tyr Gly Gly Ile Ser
(2) ANGABEN ZU SEQ. ID. NO: 16:
(i) SEQUENZKENNZEICHEN

| | |
|---|---|
| SEQUENZLÄNGE: | 15 Aminosäuren |
| ART DER SEQUENZ: | Aminosäure-Sequenz |
| MERKMAL: | Vakzine gegen HCMV |

SEQUENZBESCHREIBUNG: SEQ. ID. NO: 16:
Met Ser Ile Tyr Val Tyr Ala Leu Pro Leu Lys Met Leu Asn Ile
(2) ANGABEN ZU SEQ. ID. NO: 17:
(i) SEQUENZKENNZEICHEN

| | |
|---|---|
| SEQUENZLÄNGE: | 15 Aminosäuren |
| ART DER SEQUENZ: | Aminosäure-Sequenz |
| MERKMAL: | Vakzine gegen HCMV |

SEQUENZBESCHREIBUNG: SEQ. ID. NO: 17:
Val Tyr Ala Leu Pro Leu Lys Met Leu Asn Ile Pro Ser Ile Asn
(2) ANGABEN ZU SEQ. ID. NO: 18:
(i) SEQUENZKENNZEICHEN

| | |
|---|---|
| SEQUENZLÄNGE: | 9 Aminosäuren |
| ART DER SEQUENZ: | Aminosäure-Sequenz |
| MERKMAL: | Vakzine gegen HCMV |

SEQUENZBESCHREIBUNG: SEQ. ID. NO: 18: Ala Leu Pro Leu Lys Met Leu Asn Ile
(2) ANGABEN ZU SEQ. ID. NO: 19:
(i) SEQUENZKENNZEICHEN

| | |
|---|---|
| SEQUENZLÄNGE: | 15 Aminosäuren |
| ART DER SEQUENZ: | Aminosäure-Sequenz |
| MERKMAL: | Vakzine gegen HCMV |

SEQUENZBESCHREIBUNG: SEQ. ID. NO: 19:
His Ile Met Leu Asp Val Ala Phe Thr Ser His Glu His Phe Gly
(2) ANGABEN ZU SEQ. ID. NO: 20:
(i) SEQUENZKENNZEICHEN

| | |
|---|---|
| SEQUENZLÄNGE: | 15 Aminosäuren |
| ART DER SEQUENZ: | Aminosäure-Sequenz |
| MERKMAL: | Vakzine gegen HCMV |

SEQUENZBESCHREIBUNG: SEQ. ID. NO: 20:
Asp Val Ala Phe Thr Ser His Glu His Phe Gly Leu Leu Cys Pro
(2) ANGABEN ZU SEQ. ID. NO: 21:
(i) SEQUENZKENNZEICHEN

| | |
|---|---|
| SEQUENZLÄNGE: | 9 Aminosäuren |
| ART DER SEQUENZ: | Aminosäure-Sequenz |
| MERKMAL: | Vakzine gegen HCMV |

SEQUENZBESCHREIBUNG: SEQ. ID. NO: 21:
Val Ala Phe Thr Ser His Glu His Phe
(2) ANGABEN ZU SEQ. ID. NO: 22:
(i) SEQUENZKENNZEICHEN

| | |
|---|---|
| SEQUENZLÄNGE: | 9 Aminosäuren |
| ART DER SEQUENZ: | Aminosäure-Sequenz |
| MERKMAL: | Vakzine gegen HCMV |

SEQUENZBESCHREIBUNG: SEQ. ID. NO: 22:
Ala Phe Thr Ser His Glu His Phe Gly
(2) ANGABEN ZU SEQ. ID. NO: 23:
(i) SEQUENZKENNZEICHEN

| | |
|---|---|
| SEQUENZLÄNGE: | 15 Aminosäuren |
| ART DER SEQUENZ: | Aminosäure-Sequenz |
| MERKMAL: | Vakzine gegen HCMV |

SEQUENZBESCHREIBUNG: SEQ. ID. NO: 23:
Ala Asn Asp Ile Tyr Arg Ile Phe Ala Glu Leu Glu Gly Val Trp
(2) ANGABEN ZU SEQ. ID. NO: 24:
(i) SEQUENZKENNZEICHEN

| | |
|---|---|
| SEQUENZLÄNGE: | 15 Aminosäuren |
| ART DER SEQUENZ: | Aminosäure-Sequenz |
| MERKMAL: | Vakzine gegen HCMV |

SEQUENZBESCHREIBUNG: SEQ. ID. NO: 24:
Val Cys Ser Met Glu Asn Thr Arg Ala Thr Lys Met Gln Val Ile
(2) ANGABEN ZU SEQ. ID. NO: 25:
(i) SEQUENZKENNZEICHEN

| | |
|---|---|
| SEQUENZLÄNGE: | 15 Aminosäuren |
| ART DER SEQUENZ: | Aminosäure-Sequenz |
| MERKMAL: | Vakzine gegen HCMV |

SEQUENZBESCHREIBUNG: SEQ. ID. NO: 25:
Glu Asn Thr Arg Ala Thr Lys Met Gln Val Ile Gly Asp Gln Tyr
(2) ANGABEN ZU SEQ. ID. NO: 26:
(i) SEQUENZKENNZEICHEN

| | |
|---|---|
| SEQUENZLÄNGE: | 9 Aminosäuren |
| ART DER SEQUENZ: | Aminosäure-Sequenz |
| MERKMAL: | Vakzine gegen HCMV |

SEQUENZBESCHREIBUNG: SEQ. ID. NO: 26:
Asn Thr Arg Ala Thr Lys Met Gln Val
(2) ANGABEN ZU SEQ. ID. NO: 27:
(i) SEQUENZKENNZEICHEN

| | |
|---|---|
| SEQUENZLÄNGE: | 9 Aminosäuren |
| ART DER SEQUENZ: | Aminosäure-Sequenz |
| MERKMAL: | Vakzine gegen HCMV |

SEQUENZBESCHREIBUNG: SEQ. ID. NO: 27:
Thr Arg Ala Thr Lys Met Gln Val Ile
(2) ANGABEN ZU SEQ. ID. NO: 28:
(i) SEQUENZKENNZEICHEN

| | |
|---|---|
| SEQUENZLÄNGE: | 15 Aminosäuren |
| ART DER SEQUENZ: | Aminosäure-Sequenz |
| MERKMAL: | Vakzine gegen HCMV |

SEQUENZBESCHREIBUNG: SEQ. ID. NO: 28:
Gln Pro Phe Met Arg Pro His Glu Arg Asn Gly Phe Thr Val Leu
(2) ANGABEN ZU SEQ. ID. NO: 29:
(i) SEQUENZKENNZEICHEN

| | |
|---|---|
| SEQUENZLÄNGE: | 15 Aminosäuren |
| ART DER SEQUENZ: | Aminosäure-Sequenz |
| MERKMAL: | Vakzine gegen HCMV |

SEQUENZBESCHREIBUNG: SEO. ID. NO: 29:
Pro Leu Lys Met Leu Asn Ile Pro Ser Ile Asn Val His His Tyr
(2) ANGABEN ZU SEQ. ID. NO: 30:
(i) SEQUENZKENNZEICHEN

| | |
|---|---|
| SEQUENZLÄNGE: | 15 Aminosäuren |
| ART DER SEQUENZ: | Aminosäure-Sequenz |
| MERKMAL: | Vakzine gegen HCMV |

SEQUENZBESCHREIBUNG: SEQ. ID. NO: 30:
Leu Asn Ile Pro Ser Ile Asn Val His His Tyr Pro Ser Ala Ala
(2) ANGABEN ZU SEQ. ID. NO: 31:
(i) SEQUENZKENNZEICHEN

| | |
|---|---|
| SEQUENZLÄNGE: | 15 Aminosäuren |
| ART DER SEQUENZ: | Aminosäure-Sequenz |
| MERKMAL: | Vakzine gegen HCMV |

SEQUENZBESCHREIBUNG: SEQ. ID. NO: 31:
Glu Asp Val Pro Ser Glu Lys Leu Phe Met His Val Thr Leu Gly
(2) ANGABEN ZU SEQ. ID. NO: 32:
(i) SEQUENZKENNZEICHEN

| | |
|---|---|
| SEQUENZLÄNGE: | 15 Aminosäuren |
| ART DER SEQUENZ: | Aminosäure-Sequenz |
| MERKMAL: | Vakzine gegen HCMV |

SEQUENZBESCHREIBUNG: SEQ. ID. NO: 32:
Asp Glu Glu Glu Ala Ile Val Ala Tyr Tyr Leu Ala Thr Ala Gly
(2) ANGABEN ZU SEQ. ID. NO: 33:
(i) SEQUENZKENNZEICHEN

| | |
|---|---|
| SEQUENZLÄNGE: | 15 Aminosäuren |
| ART DER SEQUENZ: | Aminosäure-Sequenz |
| MERKMAL: | Vakzine gegen HCMV |

SEQUENZBESCHREIBUNG: SEQ. ID. NO: 33:
Glu Asn Ser Asp Gln Glu Glu Ser Glu Gln Ser Asp Glu Glu Glu

## Patentansprüche

1. Peptide bestehend aus der Sequenz (I)
R_{N} - Ala Arg Ala Lys Lys Asp Glu Leu Arg Arg Lys Met Met Tyr Met- R_{c} (I) oder Peptidderivate davon, dabei steht
R_{N} für -H oder eine Amino-Schutzgruppe oder eine weitere Aminosäure außerhalb des Peptids oder Peptidderivats,
R_{c} für -OH oder eine Carboxyl-Schutzgruppe oder eine weitere Aminosäure außerhalb des Peptids oder Peptidderivats,
wobei die Peptidderivate eine Deletion, Insertion oder Substituierung von ein, zwei oder drei Aminosäuren der Sequenz (I) aufweisen, oder die Sequenz (I) bis auf neun zusammenhängende Aminosäuren gekürzt wird, wobei die Deletion N-terminal und/oder C-terminäl ist, und
wobei die Peptidderivate im wesentlichen die Funktion des Peptides mit der Sequenz (I) oder eines der folgenden Peptide
Glu Leu Arg Arg Lys Met Met Tyr Met
Asp Glu Leu Arg Arg Lys Met Met Tyr
Asp Glu Leu Arg Arg Lys Met Met Tyr Met oder
Asp Glu Leu Arg Arg Lys Met Met Tyr Met
(jede der vorherigen Sequenzen = Referenzsequenz) besitzen, in CD8⁺ T Zellen, insbesondere von mit HCMV immunisierten Personen mit geeignetem HLA-Typ, die Produktion von Interferon-γ oder TNF-α zu induzieren.

2. Peptide oder Peptidderivate nach Anspruch 1, die die Sequenz
R_{N} - Ala Arg Ala Lys Lys Asp Glu Leu Arg Arg Lys Met Met Tyr Met- R_{c}
R_{N} - Asp Glu Leu Arg Arg Lys Met Met Tyr Met- R_{c}
R_{N} - Glu Leu Arg Arg Lys Met Met Tyr Met- R_{c}
R_{N} - Asp Glu Leu Arg Arg Lys Met Met Tyr - R_{c}
R_{N} - Asp Glu Leu Arg Arg Lys Met Met Tyr Met - R_{c}
aufweisen.

3. Peptide oder Peptidderivate nach Anspruch 1, wobei die Fragmente Nonamere sind, die darin bestehen, dass die Sequenz (I) bis auf neun zusammenhängende Aminosäuren gekürzt wird, wobei die Deletion N-terminal und/oder C-terminal ist und wobei die Funktion von wenigstens einem Peptid aus der Gruppe der Referenzsequenzen im wesentlichen von dem Nonamer dabei erfüllt wird.

4. Peptide bestehend aus der Sequenz (II).
R_{N} - Glu Phe Cys Arg Val Leu Cys Cys Tyr Val Leu Glu Glu Thr Ser- R_{c} (II) oder Peptidderivate davon, dabei steht
R_{N} für -H oder eine. Amino-Schutzgruppe oder eine weitere Aminosäure außerhalb des Peptids oder Peptidderivats,
R_{c} für -OH oder eine Carboxyl-Schutzgruppe oder eine weitere Aminosäure außerhalb des Peptids oder Peptidderivats,
wobei die Peptidderivate eine Deletion, Insertion oder Substituierung von ein, zwei oder drei Aminosäuren der Sequenz (II) aufweisen, oder die Sequenz (II) bis auf neun zusammenhängende Aminosäuren gekürzt wird, wobei die Deletion N-terminal und/oder C-terminal ist, und
wobei die Peptidderivate im wesentlichen die Funktion des Peptides mit der Sequenz (II) oder eines der folgenden Peptide
Cys Arg Val Leu Cys Cys Tyr Val Leu
Arg Val Leu Cys Cys Tyr Val Leu Glu
Val Leu Cys Cys Tyr Val Leu Glu Glu
(jede der vorherigen Sequenzen = Referenzsequenz) besitzen, in CD8⁺ T Zellen, insbesondere von mit HCMV immunisierten Personen mit geeignetem HLA-Typ, die Produktion von Interferon-γ oder TNF-α zu induzieren.

5. Peptid oder Peptidderivate nach Anspruch 4, die die Sequenz
R_{N} - Glu Phe Cys Arg Val Leu Cys Cys Tyr Val Leu Glu Glu Thr Ser- R_{c}
R_{N} - Cys Arg Val Leu Cys Cys Tyr Val Leu - R_{c}
R_{N} - Arg Val Leu Cys Cys Tyr Val Leu Glu - R_{c}
R_{N} - Val Leu Cys Cys Tyr Val Leu Glu Glu - R_{c}
aufweisen.

6. Peptide oder Peptidderivate nach Anspruch 4, wobei die Fragmente Nonamere sind, die darin bestehen, dass die Sequenz (II) bis auf neun zusammenhängende Aminosäuren gekürzt wird, wobei die Deletion N-terminal und/oder C-terminal ist und wobei die Funktion von wenigstens einem Peptid aus der Gruppe der Referenzsequenzen im wesentlichen von dem Nonamer dabei erfüllt wird.

7. Peptide oder Peptidderivate nach einem der vorherigen Ansprüche, wobei R_{N} für -H oder eine Amino-Schutzgruppe und R_{c} für -OH oder eine Carboxyl-Schutzgruppe steht.

8. Peptide oder Peptidderivate nach Anspruch 7, wobei Rₙ für -H oder eine Acylgruppe und R_{c}für -OH oder eine Iminogruppe steht.

9. Peptide oder Peptidderivate nach Anspruch 8, wobei R_{N} für -H und R_{c} für-OH steht..

10. Peptide oder Peptidderivate nach einem der vorherigen Ansprüche als Medikament oder Diagnostikum.

11. Verwendung eines Peptides oder Peptidderivates nach einem der vorherigen Ansprüche zur Herstellung eines Medikaments zur Vakzinierung gegen HCMV Infektionen.

12. Verwendung eines Peptides oder Peptidderivates nach einem der Ansprüche 1 bis 9 zur Herstellung eines Diagnostikums zur Identifizierung einer Reaktion des zellulären Immunsystems gegen HCMV.

13. Verwendung eines Peptides oder Peptidderivates nacht einem der Ansprüche 1 bis 9 zur Herstellung eines Diagnostikums zur Quantifizierung einer Reaktion des zellulären Immunsystems gegen HCMV.

14. DNA, welche für eine der Aminosäuresequenzen oder deren Derivate nach einem der Ansprüche 1 bis 9 kodiert.

15. Vektoren oder Plasmide, in welche die DNA nach Anspruch 14 eingebaut ist.

16. Medikament, umfassend eine DNA, Plasmid oder Vektor nach Anspruch 14 oder 15.

## Claims

1. Peptides having the sequence (I)
R_{N} - Ala Arg Ala Lys Lys Asp Glu Leu Arg Arg Lys Met Met Tyr Met- R_{c} (I) or peptide derivatives thereof, wherein
R_{N} represents -H or an amino protective group, or one further amino acid outside the peptide or peptide derivative;
R_{c} represents -OH or a carboxy protective group, or one further amino acid outside the peptide or peptide derivative;
wherein said peptide derivatives have a deletion, insertion or substitution of one, two or three amino acids of sequence (I), or sequence (I) is truncated to nine contiguous amino acids, the deletion being an N-terminal and/or C-terminal deletion; and
wherein said peptide derivatives essentially have the functionality of the peptide of sequence (I) or of one of the following peptides:
Glu Leu Arg Arg Lys Met Met Tyr Met
Asp Glu Leu Arg Arg Lys Met Met Tyr
Asp Glu Leu Arg Arg Lys Met Met Tyr Met or
Asp Glu Leu Arg Arg Lys Met Met Tyr Met
(each of the above sequences = reference sequence); to induce the production of interferon-γ or TNF-α in CD8⁺ T cells, especially from subjects immunized with HCMV having the appropriate HLA type.

2. The peptides or peptide derivatives according to claim 1 having the sequence
R_{N} - Ala Arg Ala Lys Lys Asp Glu Leu Arg Arg Lys Met Met Tyr Met- R_{c}
R_{N} - Asp Glu Leu Arg Arg Lys Met Met Tyr Met- R_{c}
R_{N} - Glu Leu Arg Arg Lys Met Met Tyr Met- R_{c}
R_{N} - Asp Glu Leu Arg Arg Lys Met Met Tyr - R_{c}
R_{N} - Asp Glu Leu Arg Arg Lys Met Met Tyr Met - R_{c}

3. The peptides or peptide derivatives according to claim 1, wherein said fragments are nonamers formed by truncating sequence (I) to nine contiguous amino acids, wherein the deletion is an N-terminal and/or C-terminal deletion and wherein the functionality of at least one peptide from the group of reference sequences is essentially met by said nonamer.

4. Peptides consisting of the sequence (II)
R_{N} - Glu Phe Cys Arg Val Leu Cys Cys Tyr Val Leu Glu Glu Thr Ser- R_{c} (II)
or peptide derivatives thereof, wherein
R_{N} represents -H or an amino protective group, or one further amino acid outside the peptide or peptide derivative;
R_{c} represents -OH or a carboxy protective group, or one further amino acid outside the peptide or peptide derivative;
wherein said peptide derivatives have a deletion, insertion or substitution of one, two or three amino acids of sequence (II), or sequence (II) is truncated to nine contiguous amino acids, the deletion being an N-terminal and/or C-terminal deletion; and
wherein said peptide derivatives essentially have the functionality of the peptide of sequence (II) or of one of the following peptides:
Cys Arg Val Leu Cys Cys Tyr Val Leu
Arg Val Leu Cys Cys Tyr Val Leu Glu
Val Leu Cys Cys Tyr Val Leu Glu Glu
(each of the above sequences = reference sequence); to induce the production of interferon-γ or TNF-α in CD8⁺ T cells, especially from subjects immunized with HCMV having the appropriate HLA type.

5. The peptides or peptide derivatives according to claim 4 having the sequence
R_{N} - Glu Phe Cys Arg Val Leu Cys Cys Tyr Val Leu Glu Glu Thr Ser- R_{c}
R_{N} - Cys Arg Val Leu Cys Cys Tyr Val Leu - R_{c}
R_{N} - Arg Val Leu Cys Cys Tyr Val Leu Glu - R_{c}
R_{N} - Val Leu Cys Cys Tyr Val Leu Glu Glu - R_{c}

6. The peptides or peptide derivatives according to claim 4, wherein said fragments are nonamers formed by truncating sequence (II) to nine contiguous amino acids, wherein the deletion is an N-terminal and/or C-terminal deletion and wherein the functionality of at least one peptide from the group of reference sequences is essentially met by said nonamer.

7. The peptides or peptide derivatives according to any of the preceding claims, wherein R_{N} represents -H or an amino protective group and R_{c} represents -OH or a carboxy protective group.

8. The peptides or peptide derivatives according to claim 7, wherein R_{N} represents -H or an acyl group and R_{c} represents -OH or an amino group.

9. The peptides or peptide derivatives according to claim 8, wherein R_{N} represents -H and R_{c} represents -OH.

10. The peptides or peptide derivatives according to any of the preceding claims as a medicament or diagnostic agent.

11. Use of a peptide or peptide derivative according to any of the preceding claims for preparing a medicament for vaccination against HCMV infections.

12. Use of a peptide or peptide derivative according to any of claims 1 to 9 for preparing a diagnostic agent for identifying a response of the cellular immune system against HCMV.

13. Use of a peptide or peptide derivative according to any of claims 1 to 9 for preparing a diagnostic agent for quantifying a response of the cellular immune system against HCMV.

14. DNA which codes for one of the amino acid sequences or their derivatives according to any of claims 1 to 9.

15. Vectors or plasmids into which the DNA according to claim 14 has been incorporated.

16. A medicament comprising a DNA, plasmid or vector according to claim 14 or 15.

## Revendications

1. Peptides composés de la séquence (I)
R_{N} - Ala Arg Ala Lys Lys Asp Glu Leu Arg Arg Lys Met Met Tyr Met - R_{C} (I) ou dérivés peptidiques de celle-ci, où
R_{N} représente -H ou un groupe protecteur amino ou un autre acide aminé en-dehors du peptide ou du dérivé peptidique,
R_{C} représente -OH ou un groupe protecteur carboxyle ou un autre acide aminé en-dehors du peptide ou du dérivé peptidique,
dans lesquels les dérivés peptidiques présentent une délétion, une insertion ou une substitution d'un, de deux ou de trois acides aminés de la séquence (I), ou la séquence (I) est raccourcie de jusqu'à neuf acides aminés consécutifs, la délétion étant N-terminale et/ou C-terminale, et
dans lesquels les dérivés peptidiques possèdent essentiellement la fonction du peptide ayant la séquence (I) ou la séquence d'un des peptides suivants :
Glu Leu Arg Arg Lys Met Met Tyr Met
Asp Glu Leu Arg Arg Lys Met Met Tyr
Asp Glu Leu Arg Arg Lys Met Met Tyr Met ou
Asp Glu Leu Arg Arg Lys Met Met Tyr Met
(chacune des séquences précédentes = séquence de référence) dans des cellules T CD8⁺, en particulier de personnes de type HLA approprié immunisées avec un CMV humain, qui induisent la production d'interféron γ ou de TNF-α.

2. Peptides ou dérivés peptidiques selon la revendication 1, qui présentent la séquence
R_{N} - Ala Arg Ala Lys Lys Asp Glu Leu Arg Arg Lys Met Met Tyr Met - R_{C}
R_{N} - Asp Glu Leu Arg Arg Lys Met Met Tyr Met - R_{C}
R_{N} - Glu Leu Arg Arg Lys Met Met Tyr Met - R_{C}
R_{N} - Asp Glu Leu Arg Arg Lys Met Met Tyr - R_{C}
R_{N} - Asp Glu Leu Arg Arg Lys Met Met Tyr Met - R_{C}.

3. Peptides ou dérivés peptidiques selon la revendication 1, dans lesquels les fragments sont des nonamères qui consistent en ce que la séquence (I) est raccourcie de jusqu'à neuf acides aminés consécutifs, la délétion étant N-terminale et/ou C-terminale, et la fonction d'au moins un peptide issu du groupe des séquences de référence étant essentiellement remplie par le nonamère.

4. Peptides composés de la séquence (II)
R_{N} - Glu Phe Cys Arg Val Leu Cys Cys Tyr Val Leu Glu Glu Thr Ser - R_{c} (II) ou dérivés peptidiques de celle-ci, où
R_{N} représente -H ou un groupe protecteur amino ou un autre acide aminé en-dehors du peptide ou du dérivé peptidique,
R_{C} représente -OH ou un groupe protecteur carboxyle ou un autre acide aminé en-dehors du peptide ou du dérivé peptidique,
dans lesquels les dérivés peptidiques présentent une délétion, une insertion ou une substitution d'un, de deux ou de trois acides aminés de la séquence (II), ou la séquence (II) est raccourcie de jusqu'à neuf acides aminés consécutifs, la délétion étant N-terminale et/ou C-terminale, et dans lesquels les dérivés peptidiques possèdent essentiellement la fonction du peptide ayant la séquence (II) ou la séquence d'un des peptides suivants :
Cys Arg Val Leu Cys Cys Tyr Val Leu
Arg Val Leu Cys Cys Tyr Val Leu Glu
Val Leu Cys Cys Tyr Val Leu Glu Glu
(chacune des séquences précédentes = séquence de référence) dans des cellules T CD8⁺, en particulier de personnes de type HLA approprié immunisées avec un CMV humain, qui induisent la production d' interféron γ ou de TNF-α.

5. Peptides ou dérivés peptidiques selon la revendication 4, qui présentent la séquence
R_{N} - Glu Phe Cys Arg Val Leu Cys Cys Tyr Val Leu Glu Glu Thr Ser - R_{C}
R_{N} - Cys Arg Val Leu Cys Cys Tyr Val Leu - R_{C}
R_{N} - Arg Val Leu Cys Cys Tyr Val Leu Glu - R_{C}
R_{N} - Val Leu Cys Cys Tyr Val Leu Glu Glu - R_{C}.

6. Peptides ou dérivés peptidiques selon la revendication 4, dans lesquels les fragments sont des nonamères qui consistent en ce que la séquence (II) est raccourcie de jusqu'à neuf acides aminés consécutifs, la délétion étant N-terminale et/ou C-terminale, et la fonction d'au moins un peptide issu du groupe des séquences de référence étant essentiellement remplie par le nonamère.

7. Peptides ou dérivés peptidiques selon l'une des revendications précédentes, dans lesquels R_{N} représente -H ou un groupe protecteur amino et R_{C} représente -OH ou un groupe protecteur carboxyle.

8. Peptides ou dérivés peptidiques selon la revendication 7, dans lesquels R_{N} représente -H ou un groupe acyle et R_{C} représente -OH ou un groupe amino.

9. Peptides ou dérivés peptidiques selon la revendication 8, dans lesquels R_{N} représente -H et R_{C} représente -OH.

10. Peptides ou dérivés peptidiques selon l'une des revendications précédentes en tant que médicament ou agent de diagnostic.

11. Utilisation d'un peptide ou d'un dérivé peptidique selon l'une des revendications précédentes pour fabriquer un médicament destiné à la vaccination contre des infections par des CMV humains.

12. Utilisation d'un peptide ou d'un dérivé peptidique selon l'une des revendications 1 à 9 pour fabriquer un agent de diagnostic permettant d'identifier une réaction du système immunitaire cellulaire contre un CMV humain.

13. Utilisation d'un peptide ou d'un dérivé peptidique selon l'une des revendications 1 à 9 pour fabriquer un agent de diagnostic permettant de quantifier une réaction du système immunitaire cellulaire contre un CMV humain.

14. ADN qui code pour l'une des séquences d'acides aminés ou de ses dérivés selon l'une des revendications 1 à 9.

15. Vecteurs ou plasmides dans lesquels est incorporé de l'ADN selon la revendication 14.

16. Médicament comprenant un ADN, un plasmide ou un vecteur selon la revendication 14 ou 15.
